# EUROPEAN PATENT APPLICATION

(11) **EP 1 270 001 A1**
(43) Date of publication of application: **02.01.2003**
(21) Application number: 01121909.4
(22) Date of filing: 12.09.2001
(51) Int. Cl.: A61K 31/19, A61K 33/06, A61P 3/12, A61P 19/06, A61K 9/20

(54) **Pharmaceutical composition containing calcium acetate and calcium carbonate**

(30) Priority: 19.06.2001 IT MI20011284
(71) Applicant: Bilardello, Salvatore, 91025 Marsala, (Trapani) (IT)
(72) Inventor: Bilardello, Salvatore, 91025 Marsala, (Trapani) (IT)
(74) Representative: Minoja, Fabrizio, Dr.

(57) **Abstract**

A pharmaceutical composition useful for the treatment of hyperphosphatemia, in particular for decreasing the high blood concentration of phosphate which is present in uremic patients or in patients with chronic renal failure.

## Description

The present invention relates to a pharmaceutical composition useful for the treatment of hyperphosphatemia, in particular for decreasing the high blood concentration of phosphate which is present in uremic patients or in patients with chronic renal failure.

### BACKGROUND OF THE INVENTION

A major problem with uremic or chronic renal failure (C.R.F.) patients who undergo hemodialysis is represented by hyperphosphoremia, namely the accumulation of serum phosphates, which cannot be removed by dialysis.

In human body, phosphates consist of an endogenous part (about 30%) and an exogenous part introduced from diet (about 70%). A suitably planned diet for these patients and dialysis alone cannot, however, reduce or normalize phosphoremia.

High phosphoremia should be prevented in that it affects the physiological phosphorus/calcium balance. High phosphatemia involves in fact a marked decrease in serum calcium, which induces metastatic or ectopic calcifications; on the other hand, a decrease in calcemia causes PTH (Parathormone) serum levels to increase, with consequent risks of osteodystrophy.

It is therefore evident that the control of phosphoremia and PTH in nephropathic patients is of remarkable importance in order to improve their quality of life.

Phosphorus binders, known as antihyperphosphatemic agents, are used to obviate said problems. These phosphorus binders bind phosphates causing precipitation of insoluble salts, which are subsequently removed through feces. The salification reaction should take place in the gastrointestinal tract and during meals in order to attain a qualitatively and quantitatively high yield. This way, action on most exogenous phosphates, namely those introduced through diet, is ensured.

The following compounds are at present used as antihyperphosphatemics:
- Aluminium hydroxide
- Magnesium hydroxide
- Calcium carbonate
- Calcium citrate
- Calcium acetate
- Non-calcium polymer (Renagel)

These compounds, in the cationic form, have some affinity to phosphates with which they form an insoluble precipitate, with the exception of Renagel. Said compounds are not, however, free from undesired effects.

Aluminium compounds, for example, cause accumulation of this metal which causes cerebral damage (encephalopathies); calcium compounds induces hypercalcemia to various extents; magnesium compounds cause hypermagnesiemia and cathartic effects; polymers damage the intestinal bacterial flora, destroy a number of vitamins, and often induce nausea, vomit and intestinal swelling.

The use of the calcium acetate salts as phosphorus binders is extensively disclosed in US 4,870.105, in which the properties of calcium acetate, in terms of solubility, phosphorus binding and inhibition of its absorption, are compared with those of other conventional phosphorus binders.

### DISCLOSURE OF THE INVENTION

It has now surprisingly been found that a marked reduction of phosphoremia as well as an improved control of calcemia can be obtained by a pharmaceutical composition containing calcium acetate in combination with calcium carbonate. Therefore, the present invention relates to an oral solid composition containing calcium acetate as active ingredient, which is capable of binding inorganic phosphate in the gastrointestinal tract, and calcium carbonate as an excipient, whose function is to increase the disintegration rate of the composition itself.

The effects of the composition of the invention are most likely due to the addition of calcium carbonate to the calcium acetate solid form, which increases the disintegration rate of the latter, thus making the calcium ions inside the gastrointestinal tract readily available, inducing rapid formation of the calcium phosphate insoluble salt. As a consequence, the time during which calcium and phosphate ions are in contact with gastrointestinal mucosa, and therefore the absorption thereof, are reduced.

The amount of calcium carbonate capable of inducing the described effects can range from 1 to 3% on the total composition weight. It can easily be evinced that calcium carbonate contribution to phosphorus binding is negligible, whereas its action on the tablet disintegration rate, which is remarkably increased, is essential, as evidenced by the results reported in the examples.

The composition of the invention may contain, in addition to calcium acetate and calcium carbonate, pharmaceutically acceptable excipients such as lubricants, disintegrants, dyes, sweetening agents, diluents, and the like. Suitable oral solid forms comprise tablets, lozenges and granulates. According to a preferred embodiment, the composition of the invention is in the form of a tablet.

Principles and methods for the preparation of tablets or of other oral solid forms are known to those skilled in the art and are, for example, described in Remington's Pharmaceutical Sciences Handbook, Mack Pub. Co., NY, USA, XVII Ed.

Most preferred are tablets having a content in active ingredient (calcium acetate) of 500 mg.

The composition of the invention will be administered during or near meals, in order to reduce as much as possible the time necessary for the reaction between calcium and phosphate, thus preventing the absorption of the corresponding free ions.

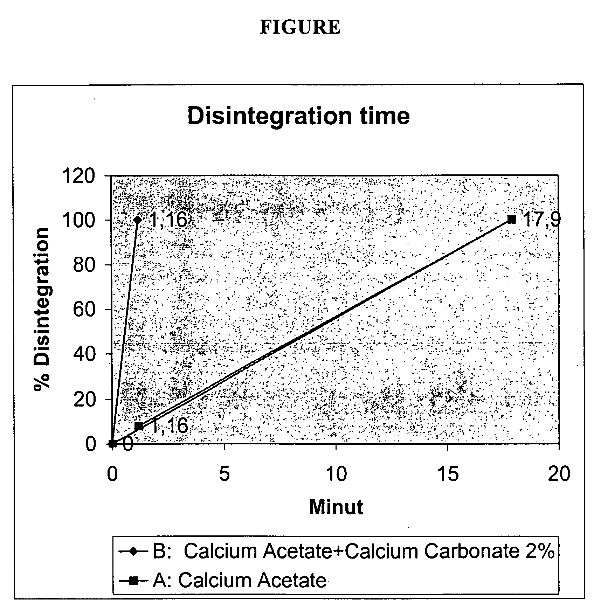

### DISCLOSURE OF THE FIGURE

Figure: disintegration time of the two compounds. Line A) is the compound calcium acetate alone, whereas line B) relates to the compound calcium acetate + 2% calcium carbonate. It is evident that at the time corresponding to 100% disintegration of compound (B), only 9% of compound (A) is disintegrated (cross mark on the line A).

The following examples illustrate the invention in greater detail.

### Example 1 - In vitro disintegration test

Disintegration time is the time necessary for a tablet to dissolve in the stomach, i.e. to be transformed into granules or aggregates.

Disintegration time was evaluated according to the method indicated in Italian Pharmacopoeia F.U. IX.

The apparatus, Perkin - Elmer PED 213, has as main component a basket fitted with 6 glass tubes, which can be raised or lowered in a vessel containing an aqueous solution similar to gastric juices as far as the most important parameters (pH and temperature) are concerned. The glass basket allows to observe the disintegration of the tablets.

Artificial gastric juices consist of 250 ml of an aqueous solution at temperature T = 37°C and pH = 1 by addition of 0.1N HCl.

The tablet is introduced in each one of the six tubes and the basket is immersed in the described fluid. The apparatus is then operated and the complete disintegration of the tablets is observed through the glass basket.

The test is carried out 3 times for both types of acetate, i.e. the standard one and that added with carbonate.

The data are reported in Table 1 and in the Figure.

The compositions of the tablets are reported in Table 2.

**Table 1**

| (times are expressed in minutes) | | | | |
|---|---|---|---|---|
| Compound | Disint. time: 1^{st} test | Disint. time: 2^{nd} test | Disint. time: 3^{rd} test | Mean |
| Ca acetate alone | 18.3 min. | 17.5 min. | 17.9 min. | 17.9 min. |
| Ca acetate added with 1% Ca carbonate | 1.31 min. | 1.33 min. | 1.30 min. | 1.313 min. |
| Ca acetate added with 2% Ca carbonate | 1.16 min. | 1.14 min. | 1.18 min. | 1.16 min. |
| Ca acetate added with 3% Ca carbonate | 1.11 min. | 1.09 min. | 1.10 min. | 1.10 min. |

**Table 2**

| COMPOUND | COMPOSITION |
|---|---|
| Calcium acetate alone | Dry calcium acetate 500 mg, vegetable salt, maltodextrin, cellulose, rice starch, Mg stearate, silica gel, methocel E5, talc, titanium dioxide, glycerol, aspartame |
| Calcium acetate added with 1% Ca carbonate | Same composition + 1% calcium carbonate (mg 5) |
| Calcium acetate added with 2% Ca carbonate | Same composition + 2% calcium carbonate (mg 10) |
| Calcium acetate added with 3% Ca carbonate | Same composition + 3% calcium carbonate (mg 15) |
| | |

Disintegration times of the two compounds show that disintegration of calcium acetate added with 1% calcium carbonate is markedly more rapid.

### Example 2 - In vivo test

The in vivo test was carried out on 20 patients appropriately selected according to the type and severity of the disease; patients under dialysis with the same severity of chronic renal failure (CRF), 8 women and 12 men, of age ranging from 46 to 55 years, with comparable levels of serum phosphoremia and calcemia. None had previously been treated with calcium acetate, see Table 3.

Therapy consisted in the administration of 1.5 g/main meal (3 g/day) for 4 weeks. All patients received, the day before starting the treatment, a solution of electrolytes and mannitol for intestinal lavage.

All patients received similar meals, to ensure similar phosphate contents were introduced through diet, see Table 4.

Undesired effects were evaluated in terms of nausea, vomit, constipation, diarrhea, skin rush, stomach swelling or heaviness, see Table 5.

None of the patients has dropped out of therapy or suspended it.

2 Homogeneous groups of patients were tested:
group A) 4 women and 6 men, treated with calcium acetate alone.
group B) 4 women and 6 men, treated with calcium acetate added with 2% calcium carbonate.

**Table 3:**

| **Data and characteristics of the patients under treatment:** | | | | | |
|---|---|---|---|---|---|
| Sex | Number | Disease | Age | Phosphoremia mg/dl | Calcemia mg/dl |
| Men | 6 | Mean-high CRF | 50/52 | 6.5/7.5 | 8.5/9.0 |
| Men | 5 | Mean-high CRF | 55/56 | 5.5/6.5 | 8.5/9.0 |
| Men | 1 | Mean-high CRF | 47 | 8.0/8.5 | 9.0/9.5 |
| Women | 5 | Mean-high CRF | 49/51 | 6.5/7.5 | 9.5/9.5 |
| Women | 3 | Mean-high CRF | 52/53 | 6.5/7.5 | 9.0/10.0 |
| Total | 20 | | | | |
| Serum mean | | | | 7.05 | 9.15 |

Blood samples were controlled three times for serum phosphoremia and calcemia: 1^{st} control before treatment with phosphorus binders; 2^{nd} control at half cycle (2 weeks); 3^{rd} control at the end of treatment (4 weeks), see Table 6.

**Table 4.**

| **Quali-quantitative composition of the meal; phosphorus (P) intake expressed in mg, approximately 750-800 mg/day.** | | |
|---|---|---|
| Meal | Composition | Phosphorus intake (P) in mg |
| Breakfast | 1 cup of coffee or tea, 30 g of bread, butter and jam or 3 biscuits | 75 mg |
| Lunch | 50 g of pasta or rice with tomato sauce 100 g of green salad or boiled potatoes 100 g of veal or chicken 1 apple or pear or orange 75 ml of wine 250 ml of water 100 g of bread | 430 mg |
| Dinner | 30 g of ham+ 50 g of mozzarella or 100 g of sole 50 g of boiled carrots 50 g of bread 75 ml of wine 250 ml of water 1 apple or pear or orange | 290 mg |

**Table 5.**

| **Undesired effects observed in the two groups during treatment.** | | | | |
|---|---|---|---|---|
| Undesired effects | N. of cases group A | N. of cases group B | Group B % | Group A % |
| Nausea | 2 | 0 | 20 | 0 |
| Vomit | 0 | 0 | | 0 |
| Constipation | 0 | 0 | | 0 |
| Diarrhea | 1 | 0 | 10 | 0 |
| Skin rush | 1 | 0 | 10 | 0 |
| Stomach Swelling or Heaviness | 2 | 1 | 20 | 10 |

**Table 6.**

| **Phosphoremia (mg/dl) and calcemia (mg/dl) of the two groups at the beginning of treatment (1**^{**st**} **control), after two weeks (2**^{**nd**} **control) and at the end of treatment (3**^{**rd**} **control); group A: Ca acetate alone; group B: Ca acetate added with 2% carbonate.** | | |
|---|---|---|
| Controls | Phosphoremia (mg/dl) | Calcemia (mg/dl) |
| 1^{st} control group A | 7.15 | 9.12 |
| 2^{nd} control group A | 6.4 | 11.3 |
| 3^{rd} control group A | 5.8 | 11.9 |
| 1^{st} control group B | 7.1 | 9.7 |
| 2^{nd} control group B | 5.9 | 10.2 |
| 3^{rd} control group B | 5.3 | 10.5 |
| % Difference (A-B) 2^{nd} control | 7.81% | 10.78% |
| % Difference (A-B) 3^{rd} control | 8.62% | 11.76% |

## Claims

1. Oral solid composition for the treatment of hyperphosphatemia, comprising calcium acetate and an amount of calcium carbonate ranging from 1 to 3% by weight.

2. Composition as claimed in claim 1, further comprising pharmaceutically acceptable excipients.

3. Composition as claimed in claims 1-2, in the form of tablet, lozenge or granulate.

4. Composition as claimed in claim 3, wherein calcium acetate content is 500 mg.

5. The use of a mixture of calcium acetate and calcium carbonate, the latter in amounts ranging from 1 to 3% by weight of the mixture, for the preparation of an oral solid composition for the treatment of hyperphosphatemia.

6. The use as claimed in claim 5, wherein said composition is a tablet, lozenge or granulate.
